(19) Europäisches Patentamt
European Patent Office
Office européen des brevets

(11) Veröffentlichungsnummer : **0 022 551**
**B1**

(12) # EUROPÄISCHE PATENTSCHRIFT

(45) Veröffentlichungstag der Patentschrift : **07.04.82**

(51) Int. Cl.³ : **C 07 D 207/38**, A 01 N 43/36

(21) Anmeldenummer : **80103906.6**

(22) Anmeldetag : **09.07.80**

(54) **2-Dihalogenmethylen-3-halogen-3-carboalkoxy-5-oxopyrrolidine, Verfahren zu ihrer Herstellung und ihre Verwendung als fungizide, bakterizide und algizide Schädlingsbekämpfungsmittel.**

(30) Priorität : **13.07.79 DE 2928305**

(43) Veröffentlichungstag der Anmeldung : **21.01.81 (Patentblatt 81/03)**

(45) Bekanntmachung des Hinweises auf die Patenterteilung : **07.04.82 Patentblatt 82/14**

(84) Benannte Vertragsstaaten : **AT BE CH DE FR GB IT LI NL**

(56) Entgegenhaltungen : **DE - A - 2 055 075**

(73) Patentinhaber : **HOECHST AKTIENGESELLSCHAFT**
**Postfach 80 03 20**
**D-6230 Frankfurt/Main 80 (DE)**

(72) Erfinder : **Mildenberger, Hilmar, Dr.**
**Fasanenstrasse 24**
**D-6233 Kelkheim (Taunus) (DE)**
Erfinder : **Gerber, Hans-Gerd, Dr.**
**Frankfurter Strasse 202**
**D-6233 Kelkheim (Taunus) (DE)**
Erfinder : **Matterstock, Karl, Dr.**
**Lessingstrasse 38**
**D-6238 Hofheim an Taunus (DE)**
Erfinder : **Sachse, Burkhard, Dr.**
**An der Ziegelei 30**
**D-6233 Kelkheim (Taunus) (DE)**
Erfinder : **Hartz, Peter, Dr.**
**An der Ziegelei 28**
**D-6233 Kelkheim (Taunus) (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 022 551 B1

# 2-Dihalogenmethylen-3-halogen-3-carboalkoxy-5-oxopyrrolidine, Verfahren zur ihrer Herstellung und ihre Verwendung als fungizide, bakterizide und algizide Schädlingsbekämpfungsmittel

Gegenstand der Erfindung sind neue 2-Dihalogenmethylen-3-halogen-3-carboalkoxy-5-oxopyrrolidine der allgemeinen Formel I,

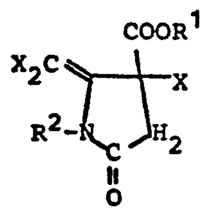

(I)

worin

$R^1$ Wasserstoff oder $(C_1$-$C_4)$-Alkyl,

$R^2$ Wasserstoff, $(C_1$-$C_4)$-Alkyl, $(C_1$-$C_4)$-Alkoxycarbonylmethyl, Cyclohexyl, Benzyl, $(C_1$-$C_4)$-Alkylphenyl, Halogenphenyl, Nitrophenyl, $(C_1$-$C_4)$-Alkoxyphenyl, Trihalogenmethylphenyl oder $(C_1$-$C_4)$-Alkoxycarbonylphenyl,

und

X Halogen bedeuten.

Bevorzugte Reste sind z.B.

$R^1 = (C_1$-$C_4)$-Alkyl,

$R^2$ = Methyl, Methoxycarbonylmethyl, 2,4-Dichlorphenyl, 3-Trifluormethylphenyl, Benzyl, 2,6-Dimethylphenyl, 2-Chlor-4-trifluormethylphenyl, 3-Chlor-4-methylphenyl, 3,5-bis-Trifluormethylphenyl, 2,3-Dimethylphenyl, 4-Ethoxycarbonylphenyl, 4-Chlorphenyl, Phenyl, 4-Methoxyphenyl, 2-Methoxyphenyl oder 4-Bromphenyl,

und

X = Cl

Folgende Verbindungen stellen eine beispielshafte Auswahl an erfindungsgemäßen Verbindungen der Formel I dar:

2-Dichlormethylen-3-chlor-3-carbomethoxy-5-oxopyrrolidin, 1-Methyl-2-dichlormethylen-3-chlor-3-carbomethoxy-5-oxopyrrolidin,
1-Benzyl-2-dichlormethylen-3-chlor-3-carbomethoxy-5-oxopyrrolidin,
1-(4-Chlorbenzyl)-2-dichlormethylen-3-chlor-3-carbomethoxy-5-oxopyrrolidin,
1-(2,4-Dichlorbenzyl)-2-dichlormethylen-3-chlor-3-carbomethoxy-5-oxopyrrolidin,
1-Cyclohexyl-2-dichlormethylen-3-chlor-3-carbomethoxy-5-oxopyrrolidin,
1-Phenyl-2-dichlormethylen-3-chlor-3-carbomethoxy-5-oxopyrrolidin,
1-(4-Methylphenyl)-2-dichlormethylen-3-chlor-3-carbomethoxy-5-oxopyrrolidin,
1-(2,6-Dimethylphenyl)-2-dichlormethylen-3-chlor-3-carbomethoxy-5-oxopyrrolidin,
1-(2-Methyl-6-ethylphenyl)-2-dichlormethylen-3-chlor-3-carbomethoxy-5-oxopyrrolidin,
1-(2,3-Dimethylphenyl)-2-dichlormethylen-3-chlor-3-carbomethoxy-5-oxopyrrolidin,
1-(2,6-Diethylphenyl)-2-dichlormethylen-3-chlor-3-carbomethoxy-5-oxopyrrolidin,
1-(4-Ethoxycarbonylphenyl)-2-dichlormethylen-3-chlor-3-carbomethoxy-5-oxopyrrolidin,
1-(4-Chlorphenyl)-2-dichlormethylen-3-chlor-3-carboethoxy-5-oxopyrrolidin,
1-(2,4-Dichlorphenyl)-2-dichlormethylen-3-chlor-3-carbomethoxy-5-oxopyrrolidin,
1-(2,6-Dichlorphenyl)-2-dichlormethylen-3-chlor-3-carbomethoxy-5-oxopyrrolidin,
1-(3,5-Dichlorphenyl)-2-dichlormethylen-3-chlor-3-carbomethoxy-5-oxopyrrolidin,
1-(4-Bromphenyl)-2-dichlormethylen-3-chlor-3-carbomethoxy-5-oxopyrrolidin,
1-(4-Fluorphenyl)-2-dichlormethylen-3-chlor-3-carbomethoxy-5-oxopyrrolidin,
1-(4-Methoxyphenyl)-2-dichlormethylen-3-chlor-3-carbomethoxy-5-oxopyrrolidin,
1-(2-Methoxyphenyl)-2-dichlormethylen-3-chlor-3-carbomethoxy-5-oxopyrrolidin,
1-(3-Trifluormethylphenyl)-2-dichlormethylen-3-chlor-3-carbomethoxy-5-oxopyrrolidin,
1-(3,5-bis-Trifluormethyl)-2-dichlormethylen-3-chlor-3-carbomethoxy-5-oxopyrrolidin,
1-(2-Chlor-4-trifluormethylphenyl)-2-dichlormethylen-3-chlor-3-carbomethoxy-5-oxopyrrolidin,
1-(3-Chlor-4-methylphenyl)-2-dichlormethylen-3-chlor-3-carbomethoxy-5-oxopyrrolidin,
1-(3-Chlor-6-methylphenyl)-2-dichlormethylen-3-chlor-3-carbomethoxy-5-oxopyrrolidin,
1-(4-Nitrophenyl)-2-dichlormethylen-3-chlor-3-carbomethoxy-5-oxopyrrolidin,
1-(2-Chlor-6-methylphenyl)-2-dichlormethylen-3-chlor-3-carbomethoxy-5-oxopyrrolidin.

Gegenstand der Erfindung ist weiterhin ein Verfahren zur Herstellung von Verbindungen der Formel I, das dadurch gekennzeichnet ist, daß man 2-Methyl-3-carboalkoxy-pyrrolinone der allgemeinen Formel II,

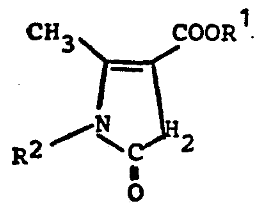

(II)

wobei $R^1$ und $R^2$ die in Formel I angegebenen Bedeutungen haben, mit Halogen umsetzt, vorzugsweise in inerten Lösungsmitteln.

Als Halogen wird bevorzugt Chlor eingesetzt.

Die Reaktionstemperatur ist nicht kritisch und kann zwischen −10 und +60 °C, vorzugsweise zwischen 5 und 30 °C liegen.

Als inerte Lösungsmittel kommen solche Verbindungen infrage, die sich unter den Reaktionsbedingungen gegenüber den Reaktanten, insbesondere gegenüber dem Halogenierungsmittel inert verhalten. Bevorzugt sind solche Lösungsmittel, die bei der Umsetzungstemperatur flüssig sind. Bevorzugte Lösungsmittel sind z.B. halogenierte Kohlenwasserstoffe, insbesondere beispielsweise Tetrachlorkohlenstoff, Methylenchlorid, Chloroform, ferner organische Säuren, insbesondere beispielsweise Essigsäure.

Erfindungsgemäß werden pro Mol Verbindung der Formel II 3 Grammäquivalente Halogen addiert.

Die Verbindungen der Formel I sind teilweise im Reaktionsgemisch schwer löslich und können durch Absaugen gewonnen werden, oder sie fallen nach dem Abdampfen des verwendeten Lösungsmittels als Öle an, die beim Verreiben mit Ether oder Benzin kristallisieren. Durch Umkristallisieren aus unpolaren Lösungsmitteln oder durch Chromatographie können sie weiter gereinigt werden.

Die als Ausgangsmaterial dienenden 2-Methyl-3-carboalkoxy-pyrrolinone der Formel II können aus Acetylbernsteinsäureester und Aminen nach bekannten Methoden hergestellt werden (vgl. z.B. W. D. Emery, Liebigs Ann. Chem. 260, 137 (1890) ; A. Cohen, J. chem. Soc., 1950, 3005 ; M. Pesson et al. Compt. rend. C 272, 478 (1971). Als Amine eignen sich hierfür prinzipiell alle primären aliphatischen und aromatischen Amine sowie auch Ammoniak.

Die erfindungsgemäßen Verbindungen der Formel I weisen eine relativ breite biocide Wirkung gegen Pilze, Bakterien und Algen auf. Sie sind besonders wirksam gegen phytopathogene Pilze, wie z.B. Botrytis cinerea, Rostpilze, Cercospora betae, Cladosporium fulvum, Fusicladium dendriticum, Piricularia oryzae und Rhizoctonia solani. Eine hervorragende Wirkung zeigen die Verbindungen gegen die der Klasse der Phycomycetes angehörenden Oomyceten, wie z.B. Phytophthora, Peronospora, Pseudoperonospora Plasmopara und Phythium.

Die Verbindungen lassen sich ferner zur Bekämpfung von nichtphytopathogenen Pilzen und Bakterien, die auf technischen Substraten wachsen und diese abbauen oder zerstören können, einsetzen. Sie erfassen u.a. Aureobasidium pullulans, Ulocladium consortiale, Aspergillus niger, Penicillium funiculosum, Poria monticola und Coniophora puteana. Ebenso werden Bakterienarten, wie z.B. Bacillus subtilis und Aerobacter aerogenes und Escherichia coli durch die beanspruchten Verbindungen in ihrem Wachstum gehemmt.

Die erfindungsgemäßen Verbindungen der Formel I sind ebenfalls wirksam gegen verschiedene Algenarten, wie z.B. Chlorella vulgaris, Anabaena flos-aquae, Spirogyra spp. und Enteromorpha spp.

Gegenstand der Erfindung sind daher auch fungizide, bakterizide und algizide Mittel, die gekennzeichnet sind durch einen Gehalt an einer Verbindung der Formel I.

Die fungiziden, bakteriziden und algiziden Mittel lassen sich in üblicher Weise, z.B. als Stäube, Spritzpulver, Beizmittel, Dispersionen, Lösungen oder Emulsionskonzentrate formulieren. Der Gehalt an Wirkstoff der Formel I beträgt in den erfindungsgemäßen Mitteln im allgemeinen etwa 2 bis 95 Gew.-%, vorzugsweise 10 bis 90 Gew.-%. Daneben enthalten die genannten Wirkstoff-Formulierungen gegebenenfalls die jeweils üblichen Haft-, Netz-, Dispergier-, Emulgier-, Penetrations-, Lösungsmittel-, Füll- und Trägerstoffe.

Die beanspruchten Verbindungen der Formel I eignen sich auch für den Einsatz im technischen Bereich, beispielsweise in Holzschutzmitteln, auf dem Anstrichfarbensektor oder als Konservierungsmittel, z.B. in Kühlschmiermitteln für die Metallbearbeitung.

Die Erfindung wird durch folgende Beispiele näher erläutert :

## A. Herstellungsbeispiele

### Beispiel 1

26,6 g (0,1 Mol) 1-(4-Chlorphenyl)-2-methyl-3-carbomethoxy-5-pyrrolinon werden in 100 ml Eisessig gelöst. Bei 10 bis 15 °C werden 21,3 g (0,3 Mol) Chlor eingeleitet. Man läßt das Chlorierungsgemisch über Nacht bei Raumtemperatur stehen, bläst dann mit Stickstoff Chlorreste aus und destilliert unter Vakuum das Lösungsmittel ab. Als Rückstand verbleiben 36 g eines hellbraunen Öls, das beim Verreiben mit Isopropyläther kristallisiert. Man saugt den Isopropylätheranteil ab und erhält 28,1 g (entsprechend 76 % d.Th.) 1-(4-Chlorphenyl)-2-dichlormethylen-3-carbomethoxy-3-chlor-5-oxopyrrolidin, Fp. : 159 °C.

Beispiele 2-30

In der Tabelle 1 sind die Reste $R^1$, $R^2$ und X in Formel I der nach den Beispielen 2 bis 30 aus den entsprechenden Verbindungen der Formel II durch Halogenierung hergestellten Verbindungen der Formel I sowie deren Schmelzpunkte (Fp.) aufgeführt. Die Beispiele werden analog dem Beispiel 1 durchgeführt.

Tabelle 1

Formel I

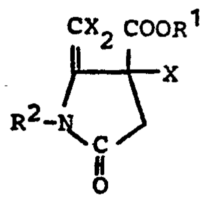

| Beispiel Nr. | $R^1$ | $R^2$ | X | Fp. [°C] |
|---|---|---|---|---|
| 2 | $CH_3$- | H- | Cl | 154 |
| 3 | $CH_3$- | $CH_3$- | Cl | 103 |
| 4 | $CH_3$- | 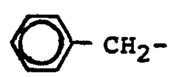 | Cl | 104 |
| 5 | $CH_3$- | 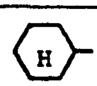 | Cl | 111 |
| 6 | $CH_3$- | 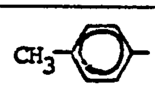 | Cl | 177 |
| 7 | $CH_3$- | 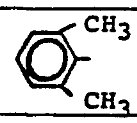 | Cl | 132 |
| 8 | $CH_3$- | 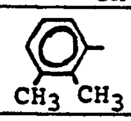 | Cl | 164 |
| 9 | $CH_3$- | 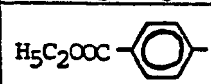 | Cl | 133 |
| 10 | $CH_3$- | 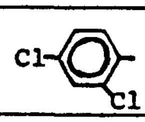 | Cl | 139 |
| 11 | $CH_3$- | 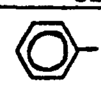 | Cl | 161 |
| 12 | $CH_3$- | 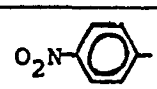 | Cl | 142 |

Tabelle 1
(Fortsetzung)

| Beispiel Nr. | $R^1$ | $R^2$ | X | Fp. [°C] |
|---|---|---|---|---|
| 13 | $CH_3$- | 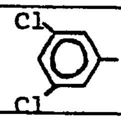 | Cl | 141 |
| 14 | $CH_3$- | 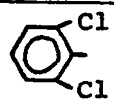 | Cl | 144 |
| 15 | $CH_3$- | 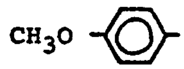 | Cl | 128 |
| 16 | $CH_3$- | 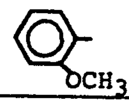 | Cl | 124 |
| 17 | $CH_3$- | 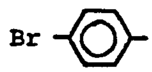 | Cl | 163 |
| 18 | $CH_3$- | 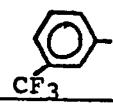 | Cl | 149 |
| 19 | $CH_3$- | 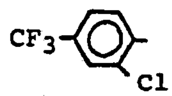 | Cl | 118 |
| 20 | $CH_3$- | 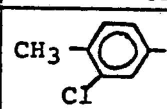 | Cl | 145 |
| 21 | $CH_3$- | 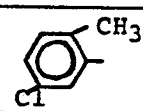 | Cl | 121 |
| 22 | $CH_3$- | 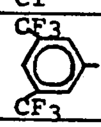 | Cl | 110 |
| 23 | $CH_3$- | 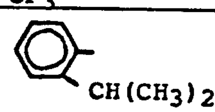 | Cl | 145 |
| 24 | $C_2H_5$- | 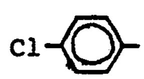 | Cl | 151 |
| 25 | $CH_3$- | 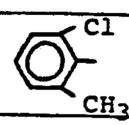 | Cl | 137 |
| 26 | $CH_3$- | 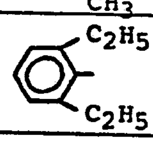 | Cl | 125 |
| 27 | $CH_3$- | 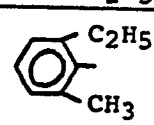 | Cl | 131 |
| 28 | $CH_3$ | 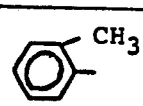 | Cl | 139 |

Tabelle 1
(Fortsetzung)

| Beispiel Nr. | $R^1$ | $R^2$ | X | Fp. [°C] |
| --- | --- | --- | --- | --- |
| 29 | $CH_3$- | $CH_3OOC-CH_2$- | Cl | 72 |
| 30 | $C_2H_5$- | 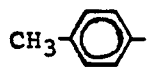 | Cl | 169 |

B. Formulierungsbeispiele

Beispiel A

Ein in Wasser leicht dispergierbares, benetzbares Pulver wird erhalten, indem man
25 Gewichtsteile Wirkstoff,
64 Gewichtsteile kaolinhaltigen Quarz als Inertstoff,
10 Gewichtsteile ligninsulfonsaures Natrium und
1 Gewichtsteil oleoylmethyltaurinsaures Natrium als Netz- und Dispergiermittel
mischt und in einer Stiftmühle mahlt.

Beispiel B

Ein Stäubemittel, das sich zur Anwendung als Pilzvertilgungsmittel gut eignet, wird erhalten, indem man
10 Gewichtsteile Wirkstoff und
90 Gewichtsteile Talkum als Inertstoff
mischt und in einer Schlagmühle zerkleinert.

Beispiel C

Ein emulgierbares Konzentrat wird erhalten aus
15 Gewichtsteilen Wirkstoff
75 Gewichtsteilen Cyclohexanon als Lösungsmittel und
10 Gewichtsteilen oxäthyliertes Nonylphenyl (10 AeO) als Emulgator.

Beispiel D

Ein Granulat wird z.B. erhalten, indem man
2-15 Gewichtsteile Wirkstoff in Toluollösung an einem inerten Granulatträgermaterial der gewünschten Korngröße, wie z.B. Attapulgit, Bimsgranulat oder Quarzsand adsorbiert und das Lösungsmittel verdunsten läßt.

C. Biologische Beispiele

In den folgenden Beispielen stehen die Buchstaben A bis G für die nachstehend genannten Vergleichsmittel:
A: Mangan-ethylen-1,2-bis-dithiocarbamat
B: N-(Trichlormethylthio)-phthalimid
C: Maneb-Zineb-Mischkomplex (Mancozeb)
D: Mergal S 40 (Kombination von Thiram und Carbendazim)
E: Mergal AT flüssig (Kombination von Ziram, Thiuram und Carbendazim)
F: Mergal CAB 40 (Kombination von Chloracetaldehyd und Natriumbisulfit)
G: Mergal AF (Kombination von Chloracetamid, Alkalifluorid und einer quaternären Ammoniumverbindung)

Beispiel I

Tomatenpflanzen (Solanum lycopersicum) der Sorte Rheinlands Ruhm werden im 3-Blattstadium mit

den in Tabelle I aufgeführten Verbindungen in den Wirkstoffkonzentrationen von 500, 250, 125 und 60 mg/Liter Spritzbrühe tropfnaß gespritzt. Als Vergleich wird das Vergleichsmittel A eingesetzt.

Nach dem Antrocknen des Spritzbelages werden die Pflanzen mit einer Zoosporangiensuspension von Phytophthora infestans inokuliert und einen Tag land tropfnaß in einer Klimakammer bei einer Temperatur von 15 °C und einer relativen Luftfeuchtigkeit von 85-95 % gestellt. Anschließend werden sie in einem Gewächshaus aufgestellt.

Nach einer Inkubationszeit von 7 Tagen werden die Pflanzen auf Befall mit Phytophthora untersucht. Der Befallsgrad wird ausgedrückt in % befallener Blattfläche, vergleichsweise zu unbehandelten, infizierten Kontrollpflanzen (= 100 % Befall). Das Ergebnis ist in der Tabelle I zusammengefaßt.

Tabelle I

| Verb. gem. Beispiel | % Phytophthorabefall bei mg Wirkstoff/Ltr Spritzbrühe | | | |
|---|---|---|---|---|
| | 500 | 250 | 125 | 60 |
| 3 | 0 | 0 | 0 - 3 | 5 |
| 29 | 0 | 0 | 0 - 3 | 5 |
| 10 | 0 | 0 | 3 - 5 | 5 |
| 18 | 0 | 0 | 3 - 5 | 5 |
| Vergleichs-mittel A | 0 | 3 | 5 | 15 |
| unbeh., infiz. Pflanzen | 100 | | | |

Beispiel II

Weinpflanzen, die aus Stecklingen der Plasmopara-anfälligen Sorte Müller-Thurgau gezogen wurden, werden im 4-Blattstadium mit wäßrigen Suspensionen der in Tabelle II aufgeführten Verbindungen in den Wirkstoffkonzentrationen von 500, 250, 125 und 60 mg/Liter Spritzbrühe tropfnaß gespritzt. Als Vergleich werden die Vergleichsmittel B und C eingesetzt.

Nach dem Antrocknen des Spritzbelages werden die Pflanzen mit einer Zoosporangiensuspension von Plasmopara viticola inokuliert und tropfnaß in eine Klimakammer bei einer Temperatur von 20 °C und einer relativen Luftfeuchtigkeit von 100 % gestellt. Nach 24 Stunden werden die infizierten Pflanzen der Klimakammer entnommen und in ein Gewächshaus mit einer Temperatur von 23 °C und einer Luftfeuchtigkeit von ca. 80-90 % gebrach.

Nach einer Inkubationszeit von 7 Tagen werden die Pflanzen angefeuchtet, über Nacht in die Klimakammer gestellt und die Krankheit zum Asubruch gebracht. Anschließend erfolgt die Befallsauswertung. Der Befallsgrad wird ausgedrückt in % befallener Blattfläche, vergleichsweise zu unbehandelten, infizierten Kontrollpflanzen (= 100 % Befall). Das Ergebnis ist in der Tabelle II zusammengefaßt.

Tabelle II

| Verb. gem. Beispiel | % Plasmopara viticola-Befall bei mg Wirkstoff/Ltr. Spritzbrühe | | | |
|---|---|---|---|---|
| | 500 | 250 | 125 | 60 |
| 4 | 0 | 0 | 0 | 0 |
| 10 | 0 | 0 | 0 | 3 - 5 |
| 7 | 0 | 0 | 0 | 3 - 5 |
| 18 | 0 | 0 | 0 - 3 | 5 |
| 19 | 0 | 0 | 0 | 0 |
| 20 | 0 | 0 - 3 | 3 | 5 |
| 22 | 0 | 0 | 0 - 3 | 5 |
| Vergleichs- B | 0 | 3 | 5 | 10 |
| mittel C | 5 | 10 | 25 | 35 |
| unbeh., infiz. Pflanzen | 100 | | | |

Beispiel III

Jeweils 0,02 ml einer Bakteriensuspension von Bacillus subtilis werden in Petrischalen auf Nährboden (Standard-I-Nähragar für Bakterien) tropfenförmig aufgebracht; dem Agar sind zuvor im flüssigen Zustand die erfindungsgemäßen Verbindungen der angegebenen Beispiele in den in Tabelle III angegebenen Konzentrationen als Wirkstoff zugesetzt worden. Als Vergleich werden die handelsüblichen quecksilberfreien Vergleichsmittel D, E, F und G eingesetzt. Die Versuchsschalen werden bei Raumtemperatur aufgestellt.

4 Tage nach der Beimpfung der Platten wird der Durchmesser der Bakterienkolonien auf dem Agar ausgemessen und die durch die Präparate hervorgerufene Wachstumshemmung, ausgedrückt in %, vergleichsweise zur Kontrolle (= beimpfter Agar ohne Wirkstoff-Zusatz = 0 % Hemmung), ermittelt. Das Ergebnis ist in der Tabelle III zusammengefaßt.

Tabelle III

| Verb. gem. Beispiel | | Hemmung in % von Bacillus subtilis bei mg Wirkstoff pro Liter Agar | | | | |
|---|---|---|---|---|---|---|
| | | 1000 | 500 | 100 | 50 | 10 |
| 3 | | 100 | 100 | 100 | 100 | 80 |
| 4 | | 100 | 100 | 100 | 100 | 50 |
| 7 | | 100 | 100 | 100 | 100 | 100 |
| 8 | | 100 | 100 | 100 | 100 | 80 |
| 9 | | 100 | 100 | 100 | 100 | 50 |
| 10 | | 100 | 100 | 100 | 100 | 50 |
| 1 | | 100 | 100 | 100 | 100 | 100 |
| 11 | | 100 | 100 | 100 | 100 | 50 |
| 15 | | 100 | 100 | 100 | 100 | 80 |
| 16 | | 100 | 100 | 100 | 100 | 80 |
| 17 | | 100 | 100 | 100 | 100 | 80 |
| Vergleichs-mittel | D | 50 | 25 | 0 | 0 | 0 |
| | E | 50 | 25 | 0 | 0 | 0 |
| | F | 100 | 50 | 0 | 0 | 0 |
| | G | 50 | 25 | 0 | 0 | 0 |
| unbeh., infiz. Agar | | 0 | | | | |

Beispiel IV

Jeweils 0,02 ml einer Sporensuspension von Ulocladium consortiale werden in Petrischalen auf Nährboden (Biomalz-Agar für Pilze) tropfenförmig aufgebracht; dem Agar sind zuvor im flüssigen Zustand die erfindungsgemäßen Verbindungen der angegebenen Beispiele in den in Tabelle IV angegebenen Konzentrationen als Wirkstoff zugesetzt worden. Als Vergleich werden die quecksilberfreien Vergleichsmittel D, E, F und G, eingesetzt. Die Versuchsschalen werden bei Raumtemperatur aufgestellt. 6 Tage nach der Beimpfung der Platten wird der Durchmesser der Pilzkolonien auf dem Agar ausgemessen und die durch die Präparate hervorgerufene Wachstumshemmung, ausgedrückt in %, vergleichsweise zur Kontrolle (= beimpfter Agar ohne Wirkstoff-Zusatz = 0 % Hemmung), ermittelt. Das Ergebnis ist in der Tabelle IV zusammengefaßt.

Tabelle IV

| Verb. gem. Beispiel | Hemmung in % von Ulocladium consortiale bei mg Wirkstoff/Ltr. Agar | | | | | |
|---|---|---|---|---|---|---|
| | 1000 | 500 | 100 | 50 | 10 | 5 |
| 3 | 100 | 100 | 100 | 100 | 90 | 80 |
| 10 | 100 | 100 | 100 | 100 | 90 | 50 |
| Vergleichsmittel D | 100 | 100 | 100 | 80 | 30 | 0 |
| E | 100 | 20 | 0 | 0 | 0 | 0 |
| F | 100 | 100 | 60 | 0 | 0 | 0 |
| G | 100 | 100 | 80 | 0 | 0 | 0 |
| unbeh., infiz. Agar | 0 | | | | | |

Beispiel V

Jeweils 0,02 ml einer Sporensuspension von Xanthomonas malvacearum werden in Petrischalen auf Nährboden (Biomalz-Agar für Pilze) tropfenförmig aufgebracht; dem Agar sind zuvor im flüssigen Zustand die erfindungsgemäßen Verbindungen der angegebenen Beispiele in den in Tabelle V angegebenen Konzentrationen als Wirkstoff zugesetzt worden. Die Versuchsschalen werden bei Raumtemperatur aufgestellt. 4 Tage nach der Beimpfung der Platten wird der Durchmesser der Bakterienkolonien auf dem Agar ausgemessen und die durch die Präparate hervorgerufene Wachstumshemmung, ausgedrückt in %, vergleichsweise zur Kontrolle (= beimpfter Agar ohne Wirkstoff-Zusatz = 0 % Hemmung), ermittelt. Das Ergebnis ist in der Tabelle V zusammengefaßt.

Tabelle V

| Verb. gem. Beispiel | Hemmung in % von Xanthomonas malvacearum bei mg Wirkstoff/Ltr. Agar | | | |
|---|---|---|---|---|
| | 250 | 125 | 60 | 30 |
| 3 | 100 | 100 | 80 | 50 |
| 29 | 100 | 100 | 90 | 50 |
| 4 | 100 | 100 | 50 | 30 |
| unbeh., infiz. Agar | 0 | | | |

Beispiel VI

Die einzellige Grünalge Chlorella vulgaris wird in einer Nährlösung (nach Döhler) in der logarithmischen Wachstumsphase mit der in der Tabelle VI aufgeführten erfindungsgemäßen Verbindung des Beispiels 4 als Wirkstoff in den in Tabelle VI angegebenen Konzentrationen behandelt. Die Behandlungen werden in 100 ml Erlenmeyerkolben mit 30 ml Algensuspension vorgenommen. Die Versuchsgefäße werden auf ein Schüttelgerät bei Dauerbewegung (ca. 75 U/min.), Dauerlicht (ca. 3 000 Lux) und Raumtemperatur aufgestellt. Nach 10 Tagen erfolgt die Bonitur. Die durch das Präparat hervorgerufene Abtötungsrate wird in % angegebenen, vergleichsweise zur Kontrolle (= Algensuspension ohne Wirkstoffzusatz = 0 % Abtötung).

Tabelle VI

| Verb. gem. Beispiel | Algizide Wirkung gegen Chlorella vulgaris; Abtötungsrate in % bei ppm Wirkstoff | | | |
|---|---|---|---|---|
| | 500 | 250 | 125 | 60 |
| 4 | 100 | 100 | 100 | 75 |
| unbeh. Algensuspension | 0 | | | |

**Ansprüche** (für die Vertragsstaaten : BE, CH, DE, FR, GB, IT, LI, NL)

1. 2-Dihalogenmethylen-3-halogen-3-carboalkoxy-5-oxopyrrolidine der allgemeinen Formel I

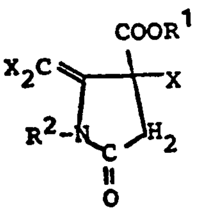

(I)

worin
$R^1$ Wasserstoff oder $(C_1$-$C_4)$-Alkyl,
$R^2$ Wasserstoff, $(C_1$-$C_4)$-Alkyl, $(C_1$-$C_4)$-Alkoxycarbonylmethyl, Cyclohexyl, Benzyl, $(C_1$-$C_4)$-Alkylphenyl, Halogenphenyl, Nitrophenyl, $(C_1$-$C_4)$-Alkoxyphenyl, Trihalogenmethylphenyl oder $(C_1$-$C_4)$-Alkoxycarbonylphenyl,
und
X Halogen bedeuten.

2. Verfahren zur Herstellung von Verbindungen der Formel I gemäß Anspruch 1, dadurch gekennzeichnet, daß man 2-Methyl-3-carboalkoxypyrrolinone der allgemeinen Formel II,

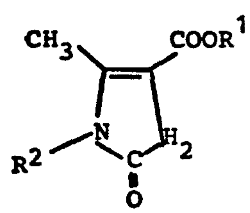

(II)

wobei $R^1$ und $R^2$ die in Formel I angegebenen Bedeutungen haben, mit Halogen umsetzt.

3. Verfahren nach Anspruch 2, dadurch gekennzeichnet, daß man die Umsetzung in Gegenwart eines inerten Lösungsmittels durchführt.

4. Verfahren nach Ansprüchen 2 und 3, dadurch gekennzeichnet, daß man als Halogen Chlor einsetzt.

5. Schädlingsbekämpfungsmittel, gekennzeichnet durch einen Gehalt an einer Verbindung der Formel I gemäß Anspruch 1 als Wirkstoff.

6. Schädlingsbekämpfungsmittel, gekennzeichnet durch einen Gehalt von 2 bis 95 Gew.-%, vorzugsweise 10 bis 90 Gew.-% an einer Verbindung der Formel I gemäß Anspruch 1 als Wirkstoff sowie üblichen Formulierungshilfsmitteln.

7. Verwendung von Verbindungen der Formel I gemäß Ansprüchen 1, 5 und 5 zur Schädlingsbekämpfung im Pflanzenschutz.

8. Verfahren zur Bekämpfung von Schadpilzen, Bakterien und Algen, im Pflanzenschutz und technischen Bereich dadurch gekennzeichnet, daß man die von ihnen befallenen Flächen, Pflanzen oder Substrate mit einer wirksamen Menge von Wirkstoffen der Formel I gemäß Ansprüchen 1, 5 und 6 in Kontakt bringt.

**Ansprüche** (für den Vertragsstaat AT)

1. Verfahren zur Herstellung von 2-Dihalogenmethylen-3-halogen-3-carboalkoxy-5-oxo-pyrrolidinen der allgemeinen Formel I,

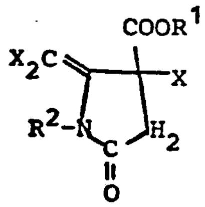

(I)

worin
$R^1$ Wasserstoff oder ($C_1$-$C_4$)-Alkyl,
$R^2$ Wasserstoff, ($C_1$-$C_4$)-Alkyl, ($C_1$-$C_4$)-Alkoxy-carbonylmethyl, Cyclohexyl, Benzyl, ($C_1$-$C_4$)-Alkylphenyl, Halogenphenyl, Nitrophenyl, ($C_1$-$C_4$)-Alkoxyphenyl, Trihalogenmethylphenyl oder ($C_1$-$C_4$)-Alkoxycarbonylphenyl,
und
X Halogen bedeuten,
dadurch gekennzeichnet, daß man 2-Methyl-3-carboalkoxypyrrolinone der allgemeinen Formel II,

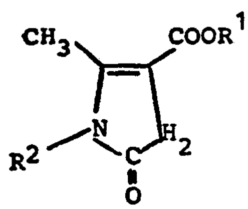

(II)

wobei $R^1$ und $R^2$ die in Formel I angegebenen Bedeutungen haben, mit Halogen umsetzt.

2. Verfahren nach Anspruch 1, dadurch gekennzeichnet, daß man die Umsetzung in Gegenwart eines inerten Lösungsmittels durchführt.

3. Verfahren nach Ansprüchen 1 und 2, dadurch gekennzeichnet, daß man als Halogen Chlor einsetzt.

4. Schädlingsbekämpfungsmittel, gekennzeichnet durch einen Gehalt and einer Verbindung der Formel I gemäß Anspruch 1 als Wirkstoff.

5. Schädlingsbekämpfungsmittel, gekennzeichnet durch einen Gehalt von 2 bis 95 Gew.-%, vorzugsweise 10 bis 90 Gew.-% an einer Verbindung der Formel I gemäß Anspruch 1 als Wirkstoff sowie üblichen Formulierungshilfsmitteln.

6. Verwendung von Verbindungen der Formel I gemäß Ansprüchen 1, 4 und 5 zur Schädlingsbekämpfung im Pflanzenschutz.

7. Verfahren zur Bekämpfung von Schadpilzen, Bakterien und Algen, im Pflanzenschutz und technischen Bereich dadurch gekennzeichnet, daß man die von ihnen befallenen Flächen, Pflanzen oder Substrate mit einer wirksamen Menge von Wirkstoffen der Formel I gemäß Ansprüchen 1, 4 und 5 in Kontakt bringt.

**Claims** (for the contracting States : BE, CH, DE, FR, GB, IT, LI, NL)

1. 2-Dihalogenomethylen-3-halogeno-3-carboalkoxy-5-oxopyrrolidines of the formula I,

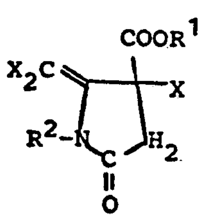

(I)

wherein
$R^1$ is hydrogen or $C_1$-$C_4$-alkyl,
$R^2$ is hydrogen, $C_1$-$C_4$-alkyl, $C_1$-$C_4$-alkoxycarbonylmethyl, cyclohexyl, benzyl, $C_1$-$C_4$-alkylphenyl,

halogenophenyl, nitrophenyl, $C_1$-$C_4$-alkoxyphenyl, trihalogenomethylphenyl or $C_1$-$C_4$-alkoxycarbonylphenyl
and
X is halogen.

2. Process for the manufacture of compounds of the formula I as claimed in Claim 1, which comprises reacting 2-methyl-3-carboalkoxypyrrolinones of the formula II,

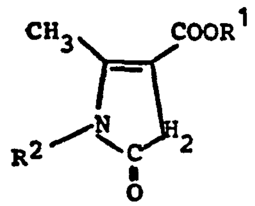

(II)

wherein $R^1$ and $R^2$ are as defined in formula I, with halogen.

3. Process as claimed in Claim 2, which comprises carrying out the reaction in the presence of an inert solvent.

4. Process as claimed in Claims 2 and 3, which comprises using chlorine as the halogen.

5. Pesticidal composition characterized by a content of a compound of the formula I as claimed in Claim 1 as the active substance.

6. Pesticidal composition characterized by a content of from 2 to 95 weight %, preferably of from 10 to 90 weight %, of a compound of the formula I as claimed in Claim 1 as the active substance, and of the usual formulation auxiliaries.

7. Use of compounds of the formula I as claimed in Claims 1, 5 and 6 for combating pests in plant protection.

8. Process for combating fungi, bacteria and algae in plant protection and in technical substrates, which comprises contacting the areas, plants or substrates infected therewith with an effective amount of active substances of the formula I as claimed in Claims 1, 5 and 6.

**Claims** (for the contracting State AT)

1. Process for the manufacture of 2-Dihalogenomethylene-3-halogeno-3-carboalkoxy-5-oxopyrrolidines of the formula I,

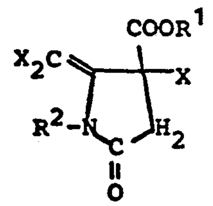

(I)

wherein
$R^1$ is hydrogen or $C_1$-$C_4$-alkyl,
$R^2$ is hydrogen, $C_1$-$C_4$-alkyl, $C_1$-$C_4$-alkoxycarbonylmethyl, cyclohexyl, benzyl, $C_1$-$C_4$-alkylphenyl, halogenophenyl, nitrophenyl, $C_1$-$C_4$-alkoxyphenyl, trihalogenomethylphenyl or $C_1$-$C_4$-alkoxycarbonylphenyl
and
X is halogen,
which comprises reacting 2-methyl-3-carboalkoxypyrrolinones of the formula II,

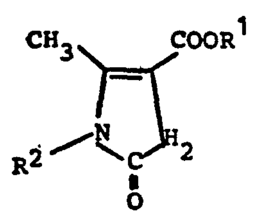

(II)

wherein $R^1$ and $R^2$ are as defined in formula I, with halogen.

2. Process as claimed in Claim 1, which comprises carrying out the reaction in the presence of an inert solvent.

3. Process as claimed in Claims 1 and 2 which comprises using chlorine as the halogen.

4. Pesticidal composition characterized by a content of a compound of the formula I as claimed in Claim 1 as the active substance.

5. Pesticidal composition characterized by a content of from 2 to 95 weight %, preferably of from 10 to 90 weight %, of a compound of the formula I as claimed in Claim 1 as the active substance, and of the usual formulation auxiliaries.

6. Use of compounds of the formula I as claimed in Claims 1, 4 and 5 for combating pests in plant protection.

7. Process for combating fungi, bacteria and algae in plant protection and in technical substrates, which comprises contacting the areas, plants or substrates infected therewith with an effective amount of active substances of the formula I as claimed in Claims 1, 4 and 5.

**Revendications** (pour les Etats contractants : BE, CH, DE, FR, GB, IT, LI, NL)

1. 2-Dihalogénométhylène-3-halogéno-3-alcoxycarbonyl-5-oxopyrrolidines de formule générale I

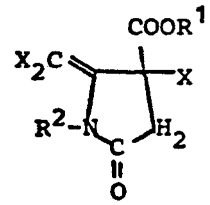

(I)

dans laquelle

$R^1$ représente un atome d'hydrogène ou un alkyle en $C_1$ à $C_4$,

$R^2$ représente un atome d'hydrogène, un alkyle en $C_1$ à $C_4$, un alcoxy ($C_1$-$C_4$) carbonylméthyle, un cyclohexyle, un benzyle, un alkyl ($C_1$-$C_4$) phényle, un halogénophényle, un nitrophényle, un alcoxy ($C_1$-$C_4$) phényle, un trihalogénométhylphényle ou un alcoxy ($C_1$-$C_4$) carbonylphényle

et

X représente un halogène.

2. Procédé de préparation de composés de formule générale I selon la revendication 1, caractérisé en ce que l'on fait réagir des 2-méthyl-3-alcoxycarbonyl-pyrrolinones de formule générale II

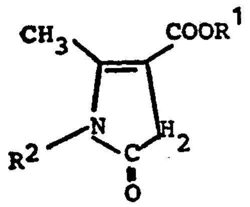

(II)

dans laquelle $R^1$ et $R^2$ ont les significations données à propos de la formule I, avec un halogène.

3. Procédé selon la revendication 2, caractérisé en ce que l'on effectue la réaction en présence d'un solvant inerte.

4. Procédé selon l'une quelconque des revendications 2 et 3, caractérisé en ce que l'on met en jeu le chlore en tant qu'halogène.

5. Agents phytosanitaires caractérisés en ce qu'ils contiennent un composé de formule I selon la revendication 1, en tant que matière active.

6. Agents phytosanitaires caractérisés en ce qu'ils contiennent de 2 à 95 % en poids, de préférence de 10 à 90 % en poids, d'un composé de formule I selon la revendication 1, en tant que matière active, ainsi que des auxiliaires de formulation habituels.

7. Utilisation de composés de formule I selon l'une quelconque des revendications 1, 5 et 6 pour combattre des déprédateurs dans le cadre de la protection de plantes.

8. Procédé pour combattre des champignons nuisibles, des bactéries et des algues tant dans le cadre de la protection des plantes que dans le domaine technique, procédé caractérisé en ce que l'on met les surfaces, plantes ou substrats attaqués par ces champignons nuisibles, bactéries et algues, au contact d'une quantité efficace de matières actives de formule I selon l'une quelconque des revendications 1, 5 et 6.

**Revendications** (pour l'Etat contractant AT)

1. Procédé de préparation de 2-dihalogénométhylène-3-halogéno-3-alcoxycarbonyl-5-oxopyrrolidines de formule générale I

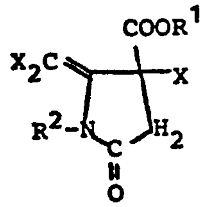

(I)

dans laquelle

$R^1$ représente un atome d'hydrogène ou un alkyle en $C_1$ à $C_4$,

$R^2$ représente un atome d'hydrogène, un alkyle en $C_1$ à $C_4$, un alcoxy ($C_1$-$C_4$) carbonylméthyle, un cyclohexyle, un benzyle, un alkyl ($C_1$-$C_4$) phényle, un halogénophényle, un nitrophényle, un alcoxy ($C_1$-$C_4$) phényle, un trihalogénométhylphényle ou un alcoxy ($C_1$-$C_4$) carbonylphényle

et

X représente un halogène,

procédé caractérisé en ce qu'on fait réagir des 2-méthyl-3-alcoxycarbonyl-pyrrolinones de formule générale II

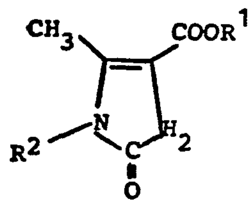

(II)

dans laquelle $R^1$ et $R^2$ ont les significations données à propos de la formule I, avec un halogène.

2. Procédé selon la revendication 1, caractérisé en ce qu'on effectue la réaction en présence d'un solvant inerte.

3. Procédé selon l'une quelconque des revendications 1 et 2, caractérisé en ce qu'on met en jeu le chlore en tant qu'halogène.

4. Agents phytosanitaires caractérisés en ce qu'ils contiennent un composé de formule I selon la revendication 1, en tant que matière active.

5. Agents phytosanitaires caractérisés en ce qu'ils contiennent de 2 à 95 % en poids, de préférence de 10 à 90 % en poids, d'un composé de formule I selon la revendication 1, en tant que matière active, ainsi que des auxiliaires de formulation habituels.

6. Utilisation de composés de formule I selon l'une quelconque des revendications 1, 4 et 5 pour combattre des déprédateurs dans le cadre de la protection de plantes.

7. Procédé pour combattre des champignons nuisibles, des bactéries et des algues tant dans le cadre de la protection des plantes que dans le domaine technique, procédé caractérisé en ce qu'on met les surfaces, plantes ou substrats attaqués par ces champignons nuisibles, bactéries et algues, au contact d'une quantité efficace de matières actives de formule I selon l'une quelconque des revendications 1, 4 et 5.